# EUROPÄISCHE PATENTSCHRIFT

(11) **EP 1 646 876 B1**
(45) Veröffentlichungstag und Bekanntmachung des Hinweises auf die Patenterteilung: **22.06.2011**
(21) Anmeldenummer: 04740826.5
(22) Anmeldetag: 08.07.2004
(51) Int. Cl.: G01N 33/80, G01N 33/558

(54) **VORRICHTUNG UND VERFAHREN ZUR SIMULTANEN BESTIMMUNG VON BLUTGRUPPENANTIGENEN**
DEVICE AND METHOD FOR SIMULTANEOUSLY IDENTIFYING BLOOD GROUP ANTIGENS
DISPOSITIF ET PROCEDE DE DETERMINATION SIMULTANEE D'ANTIGENES DE GROUPES SANGUINS

(30) Priorität: 09.07.2003 DE 10330982
(43) Veröffentlichungstag der Anmeldung: 19.04.2006
(73) Patentinhaber: Medion Diagnostics AG, 3186 Düdingen (CH)
(72) Erfinder: SCHWIND, Peter, CH-1700 Fribourg (CH); LÖSTER, Klemens, 16562 Bergfelde (DE)
(74) Vertreter: Bublak, Wolfgang
(86) Internationale Anmeldenummer: PCT/EP2004/007536
(87) Internationale Veröffentlichungsnummer: WO 2005/005991

(56) Entgegenhaltungen:
- EP-A- 0 296 724
- WO-A-97/32213
- GB-A- 2 250 342

## Beschreibung

Die Erfindung betrifft eine Vorrichtung für Lateral-Diagonal-Fluss Multiparameter Tests, insbesondere auf dem Gebiet der Blutgruppenserologie, zum gleichzeitigen, qualitativen oder quantitativen Bestimmen mehrerer Analyten in einer flüssigen Probe, wobei die Analyten Blutgruppenantigene bzw. -antigen-Epitope sind, umfassend eine Membran mit einer Aufgabezone zum Auftragen der flüssigen Probe, mindestens zwei Indikatorzonen, die mit dem/den Analyten in Wechselwirkung treten können und mindestens einem Absorptionsbereich, welcher die Flüssigkeit nach Passieren der Indikatorzonen aufnimmt, wobei die Indikatorzonen zwischen der Aufgabezone und einem Absorptionsbereich liegen, dadurch gekennzeichnet, dass die Fließrichtungen von der Aufgabezone durch die jeweiligen Indikatorzonen zu einem Absorptionsbereich (Fließspuren) im Wesentlichen parallel sind und mindestens zwei unterschiedliche Fließspuren vorliegen, wobei die Indikatorzonen so angeordnet sind, dass die Probeflüssigkeit pro Fließspur nicht mehr als eine Indikatorzone durchströmt, und die Indikatorzonen Antikörper bzw. Antikörperfragmente gegen Blutgruppenantigene und/oder Lektine bzw. Fragmente davon gegen Blutgruppenantigene umfassen.

Die Erfindung betrifft weiter ein Verfahren zur Bestimmung mehrerer Analyten in einer flüssigen Probe, umfassend das Auftragen der Probe auf die Aufgabezone einer Membran der erfindungsgemäßen Vorrichtung, wobei diese Probe in ausreichender Menge vorliegt, um die Probenflüssigkeit dazu zu veranlassen, in Richtung Absorptionsbereich durch die Indikatorzonen zu fließen und um die Analyten oder ihre Derivate in der Probenflüssigkeit dazu zu veranlassen, in den Indikatorzonen einen Komplex zu bilden, insbesondere zur simultanen Bestimmung von Blutgruppenantigenen.

In der blutgruppenserologischen Diagnostik werden allgemein Parameter nachgewiesen, die besonders im Zusammenhang mit Transfusionen bzw. dem Morbus Hämolyticus Neonatorum von Bedeutung sind. Dabei handelt es sich unter anderem um den Nachweis von Antigenen auf der Oberfläche der Erythrozyten, die für die Blutgruppen charakteristisch sind. Weitere wichtige Antigensysteme befinden sich auch auf Thrombozyten, Granulozyten, Lymphozyten, die ebenfalls bei Transfusion und/oder Transplantation eine Rolle spielen.

Bekanntermaßen werden zur Bestimmung der Blutgruppenantigene die Erythrozyten der zu testenden Person (Spender oder Empfänger) mit Reagenzien, welche blutgruppenspezifische Antikörper enthalten, zusammengebracht. Üblicherweise handelt es sich um Flüssigkeitstests, bei denen durch Mischen einer Erythrozytenhaltigen Probe mit einer Probe, welche Antikörper enthält, die gegen ein bestimmtes Blutgruppenmerkmal gerichtet sind, ein Testansatz hergestellt wird. Der Testansatz wird dann über einen definierten Zeitraum und unter definierten Bedingungen inkubiert und nach Abschluss der Inkubation oder direkt oder nach einem Zentrifugationsschritt entweder visuell oder mit optischen Methoden auf eine eventuelle Agglutination oder Adsorption der Erythrozyten überprüft. Die vorherrschende Endpunktmessung in der Blutgruppenserologie ist nach wie vor die Hämagglutination. Für jede zu bestimmende Blutgruppe muss ein eigener Ansatz pipettiert werden, d. h. z. B. die Bestimmung der 9 wichtigsten Blutgruppen A, B, D, C, c, E, e, Cw und K erfordert ohne Kontrolle 9 getrennte Ansätze.

Lateral-Fluss-Tests finden heute vielfach Anwendung als Schnelltests, z. B. als Schwangerschaftstests, zur Bestimmung von Infektionsmarkern oder als Drogenscreen. Eine Lateral-Fluss-Test-Anordnung besteht bekanntermaßen aus einem festen Träger, auf dem eine Aufgabezone für die zu untersuchende Probe aufgebracht ist, eine Trennmembran, auf der Bindungselemente, z. B. Fängerantikörper bzw. -antigene gebunden sind und auf der sich Bindungsreaktionen nachweisen lassen und ein saugfähiger Absorptionsbereich, der die zu untersuchende Probe durch die Trennmembran fließen lässt.

Testmembranen herkömmlicher Lateral-Fluss-Tests werden in der Regel mit chromatographie-ähnlicher Auftrennung beschrieben. Der Analyt in der Probe bindet spezifisch an die in einer Membran befestigten Bindungselemente, die in der Regel in hintereinanderliegenden bzw. übereinanderstehenden Banden angeordnet als Indikatorzonen vorliegen. Der Bindungskomplex wird durch Indikator-partikel sichtbar gemacht, welche in der Regel in einem Konjugat-Freisetzungs-Pad eingetrocknet in der Anordnung bereits vorliegen. Das Konjugat-Freisetzungs-Pad ist typischerweise zwischen Aufgabezone und Membran angebracht. Die vorbeschichteten farbigen Indikatorpartikel sind beispielsweise mit einem gegen den gesuchten Analyten gerichteten Antikörper beschichtet.

Das übliche Lateral-Fluss-Test-Format ist das eines sogenannten "Sandwich-Assays", bei dem sowohl die Indikatorzone als auch die Indikatorpartikel mit gegen den gesuchten Analyten gerichteten Liganden, normalerweise ein Antikörper, belegt sind. Dabei ist der Ligand (Bindungselement) an die Membran immobilisiert. Das Detektor-Reagenz, normalerweise ein Antikörper, welcher an gefärbte Polystyrol-Partikel oder kolloidale Metalle gebunden ist, ist im Konjugat-Freisetzungs-Pad auswaschbar deponiert. Dieser Bindungskomplex dient als Indikatorpartikel. Nach Auftragen der zu untersuchenden Probe benetzt diese sehr schnell das Konjugat-Freisetzungs-Pad, wodurch die Indikatorpartikel mobilisiert werden. Die Indikatorpartikel migrieren mit der Flüssigkeitsfront entlang der porösen Membran. Ein in der Probe befindlicher Analyt wird durch den Antikörper, der an das Indikatorpartikel gekoppelt ist, gebunden. Wenn die Probe die Indikatorzone passiert, wird der Analyt/Indikatorpartikel-Komplex in der Indikatorzone durch Reaktion des Analyten mit dem in der Indikatorzone gebundenen Antikörper immobilisiert, was zu einem sichtbaren Signal führt.

Ein weiteres bekanntes Testformat für kleine Analyten mit nur einer einzigen antigenen Determinante, die nicht gleichzeitig zwei Antikörper binden kann, ist der sogenannte "Kompetitions-Assay". Das an die Indikatorpartikel gebundene Detektor-Reagenz ist normalerweise ein dem Analyten identisches oder analoges Molekül. Die Indikatorpartikel sind im Konjugat-Freisetzungs-Pad deponiert. Die Indikatorpartikel migrieren mit der Flüssigkeitsfront entlang der porösen Membran. Wenn die Probe, die Analyt enthält, und die Indikatorpartikel (die effektiv ebenfalls Analyt enthalten) die Indikatorzone passieren, bindet ein Teil der Analytmoleküle in der Probe und ein Teil der Indikatorpartikel. Je mehr Analyt sich in der Probe befindet, umso effektiver wird er mit der Bindung der Indikatorpartikel kompetieren, umso schwächer wird das Signal.

Bekanntermaßen sind diese Indikatorpartikel überwiegend aus kolloidalem Gold oder aus Polystyrol, die mit dem Fachmann bekannten Methoden hergestellt und beschichtet werden. In den typischen Lateral-Fluss-Tests Formaten werden die Analyten indirekt bestimmt. Unter direkter Bestimmung eines Analyten wird hier verstanden, dass der Analyt bereits an das Indikatorpartikel (z. B. Erythrozyt) natürlich gebunden ist. In dem gebräuchlicheren Fall der indirekten Bestimmung des Analyten enthält die zu testende Probe in der Regel eine nicht zellulär gebundene, z. B. plasmatische Komponente als Analyten und es werden neben der zu testenden Probe zwei Reagenz-Komponenten benötigt, nämlich Indikatorpartikel und Bindungselement. Bei der indirekten Bestimmung bindet der Analyt zunächst an die aus dem Konjugat-Freisetzungs-Pad herausgelösten Indikator-Partikel, bevor dieser Komplex dann durch eine zweite Reaktion mit dem Bindungselement in den Indikatorzonen immobilisiert wird.

Bei der Verwendung herkömmlicher Lateral-Fluss-Tests mit Erythrozyten als Indikatorpartikeln, die die zu bestimmenden Analyten, beispielsweise Blutgruppen-spezifische Antigene, gebunden haben, werden bislang in den Indikatorzonen Antikörper gegen korrespondierende Blutgruppenantigene als Bindungselemente in hintereinanderliegenden bzw. übereinanderstehenden Banden nur einer Fließspur angeordnet, wie zum Beispiel anti-A, anti-B gegen die Blutgruppen-Antigene A bzw. B oder Antikörper gegen Antigene des Rh Blutgruppensystems. Dabei weisen herkömmliche Lateral-Fluss-Tests den Nachteil auf, dass die an die Antikörper gebundenen Erythrozyten eine Flussbarriere für die weiter zu untersuchenden Analyte, beispielsweise weitere Zell-assoziierte Antigene, in einer Probe bilden. Durch Agglutination oder Adsorption von Zellen in einer proximal zur Aufgabezone liegenden Bande von Bindungselementen können sich weitere Analyten, insbesondere assoziiert an Zellen bzw. Zellfragmente, in der zu untersuchenden Probe nicht weiter ungehemmt und sichtbar auftrennen und können folglich nicht eindeutig bzw. vollständig nachgewiesen werden. Dies kann z. B. bei einer Person, die Blutgruppe AB Rh D positiv ist, zu einer Abschwächung bzw. Eliminierung der B- und der D-Bande führen, was zu einer Fehlinterpretation im Sinne von Blutgruppe A Rh negativ führen könnte. Bislang konnten deshalb speziell in der blutgruppenserologischen Diagnostik keine Lateral-Fluss-Tests mit mehr als einer Indikatorzone angewendet werden. Für die Messung mehrerer, insbesondere zellulär gebundener und plasmatischer, Blutgruppenparameter müssen bislang Einzelparameter-Tests separat durchgeführt werden.

Aufgabe der Erfindung ist es, die im Hinblick auf den Stand der Technik angeführten Nachteile, insbesondere die der hintereinanderliegenden bzw. überlagernden Indikator- bzw. Nachweiszonen herkömmlicher Lateral-Fluss-Tests, für eine gleichzeitige Messung verschiedener Proben-Parameter, insbesondere von zellulären und plasmatischen Parametern, zu überwinden.

Die Aufgabe wird erfindungsgemäß gelöst zum einen durch eine Vorrichtung zum gleichzeitigen, qualitativen oder quantitativen Bestimmen eines oder mehrerer Analyten in einer flüssigen Probe oder mehrerer flüssiger Proben, die eine Membran umfasst mit einer Aufgabezone zum Auftragen der flüssigen Probe, mindestens zwei Indikatorzonen, die mit den/dem Analyten bzw. mit denen Analyten in Wechselwirkung treten können und mindestens einem Absorptionsbereich, welcher die Flüssigkeit nach Passieren der Indikatorzonen aufnimmt, wobei die Indikatorzonen zwischen der Aufgabezone und einem Absorptionsbereich liegen, dadurch gekennzeichnet, dass die Fließrichtungen von der Aufgabezone durch die jeweiligen Indikatorzonen zu einem Absorptionsbereich, welche Fließspuren darstellen, im Wesentlichen parallel sind und mindestens zwei unterschiedliche Fließspuren vorliegen, und die Indikatorzonen Antikörper bzw. Antikörperfragmente gegen Blutgruppenantigene und/oder Lektine bzw. Fragmente davon gegen Blutgruppenantigene umfassen.

Die Indikatorzonen der erfindungsgemäßen Vorrichtung befinden sich auf der Membran und umfassen Bindungselemente, die den/die zu bestimmenden Analyten/Analyte in der/den Proben abfangen bzw. binden. In den Indikatorzonen werden die Bindungsreaktionen zwischen Analyt und Bindungselement nachgewiesen.

In einer Ausführungsform der Erfindung sind die Indikatorzonen so angeordnet, dass die Probenflüssigkeit pro Fließspur nicht mehr als eine Indikatorzone durchströmt. Beispielhaft sind die Indikatorzonen versetzt auf der Membran angeordnet. Die Anordnung der Indikatorzonen ist dabei vorzugsweise in einer von proximal nach distal oder umgekehrt diagonal verlaufenden Reihe ausgestaltet. Besondere Ausführungsformen sind V-förmig, W-, M-, oder N-förmig oder umgekehrt V-förmig, W-, M-, oder N-förmig ausgestaltet. In einer weiteren Ausführungsform sind die Indikatorzonen parallel nebeneinander versetzt in einer linearen Reihe angeordnet.

Die Einführung parallel versetzter Indikatorzonen macht eine Multiparametertestung mit Erythrozyten als Indikatorpartikeln in einer lateralen Anordnung erst möglich. Die besonders bevorzugte Ausführungsform einer diagonalen Anordnung hat den Vorteil, dass die Bezeichnung der Ergebnisse besonders praktisch und leicht ablesbar auf die erfindungsgemäße Anordnung aufgebracht werden kann, da jeder nachzuweisende Parameter eine definierte X- und Y-Position aufweist, betrachtet man die Anordnung der erfindungsgemäßen Vorrichtung als ein Koordinatensystem mit Ordinate (Ebene der Fließrichtung) und Abszisse (Ebene der Auftragszone).

Die Indikatorzonen umfassen Antikörper bzw. Antikörperfragmente und/oder Lektine bzw. Fragmente davon, die die zu bestimmenden Blutgruppenantigene und damit die sie tragenden Zellen in der Probe abfangen bzw. binden. Als bevorzugte Bindungselemente werden Antikörper bzw. Antikörperfragmente und/oder Lektine bzw. Fragmente davon gegen Antigene aller denkbaren Blutgruppensysteme in den Indikatorzonen an der porösen Membran angebracht. Vorzugsweise wird in einer Indikatorzone, vorzugsweise in einer distal zu allen übrigen Indikatorzonen gelegenen Indikatorzone, ein Kontroll-Bindungselement (Kontrolle = ctl) angebracht, das den Durchfluss der Probe durch die Indikatorzonen positiv anzeigt. Das Kontroll-Bindungselement ist vorzugsweise ein polyklonaler anti-Erythrozyten-Antikörper.

In einer bevorzugten Ausführungsform umfasst dabei jeweils eine Indikatorzone ein Bindungselement, vorzugsweise einen Antikörper bzw. ein Antikörperfragment, gegen einen zu untersuchenden Analyten. Bevorzugte Ausführungsformen von Antikörpern bzw. Antikörperfragmenten und/oder Lektinen bzw. Fragmenten davon in den Indikatorzonen sind Antikörper bzw. Lektine gegen Antigene des AB0-Blutgruppensystem des Rh-, Kell-, Lewis- Hh-, Duffy- Kidd, MNS-, Lutheran-, P-Systems. Weiter bevorzugt als Bindungselemente der Indikatorzonen sind Antikörper gegen Antigene der Blutgruppensysteme Diego, Yt, Scianna, Dombrock, Colton, Chido/Rodgers, Gerbich, Cromer, Knops, Landsteiner-Wiener, Xg, Kx, Indian, Ok, Raph, John Milton Hagen, Langereis, und/oder Sid. Eine besonders bevorzugte Ausführungsform der erfindungsgemäßen Vorrichtung umfasst Indikatorzonen mit den Bindungselementen anti-A, -B, -AB, -D, -D, -C, - c, -E, -e, -Cw und/oder -K-Antikörper bzw. deren Antikörperfragmente, wobei die beiden anti-D zwei verschiedene Antikörper bzw. deren Antikörperfragmente sind. Insbesondere bei Patienten, Schwangeren oder Neugeborenen sind dies vorzugsweise monoklonale Antikörper der IgM Klasse, die die D^{VI}-Kategorie nicht erfassen. Bei Spendern ist dies vorzugsweise ein Antikörper, der die D^{VI}-Kategorie erfasst und ein Antikörper, der die D^{VI}-Kategorie nicht erfasst.

Durch die erfindungsgemäße Vorrichtung muss für die Blutgruppenbestimmung nicht mehr für jede einzelne Bestimmung separat pipettiert werden, sondern an einer Probe können gleichzeitig eine große Anzahl gewünschter Antigene zu untersuchender Blutgruppensysteme, beispielsweise die wichtigsten Blutgruppenmerkmale der Blutgruppensysteme AB0, Rh und Kell (A, B, AB, D, C, c, E, e, Cw, K) auf einmal bestimmt werden. Dies stellt eine außerordentliche Rationalisierung der Arbeitsabläufe dar. Auch die Ablesung der in diagonaler Anordnung dargestellten Ergebnisse ist wesentlich günstiger. Ferner lassen sich in der erfindungsgemäßen Vorrichtung beispielsweise AB0- und Rh-Eigenschaften in einer Vorrichtung nebeneinander bestimmen und ablesen. Die Zuordnung der Ergebnisse zu dem betreffenden Patienten ist erleichtert. Das zweidimensionale, flächige Ergebnis sowie der stabile Endpunkt der Reaktion begünstigen sowohl die Ablesung mit dem bloßen Auge als auch eine automatisierte Ablesung der Ergebnisse mit gängigen Bildanalyseverfahren, wie z. B. CCD-Kameras. Der Arbeitsaufwand ist vermindert, selbst bei manueller Abarbeitung. Die erfindungsgemäße Vorrichtung führt zudem zu einer Reduktion der Umweltbelastung und zu kostengünstigen Effekten. Selbst in Notsituationen mit Zeitdruck kann in kurzer Zeit in einer einzigen Versuchsanordnung beispielsweise eine komplette AB0-Blutgruppe-/Rh-Untergruppen-Bestimmung durchgeführt werden. Produktionstechnisch hat der Lateral-Diagonal-Fluss-Aufbau wesentliche Vorteile gegenüber dem Stand der Technik, indem es zu einem erheblich geringeren Verbrauch der verwendeten Reagenzien kommt und durch die Bereitstellung einer Vielzahl von Testparametern in einer einzigen Vorrichtung.

Durch die erfindungsgemäße Vorrichtung wird ein Lateral-Fluss-Test, insbesondere für die blutgruppenserologische Diagnostik, bereitgestellt, mit dem Erythrozyten als Indikatorpartikel verwendet werden und in einem Testansatz gleichzeitig mehrere zelluläre, insbesondere erythrozytäre Antigene bzw. Antigen-Epitope, plasmatische Parameter und/oder Blutzelleigenschaften, insbesondere aus Vollblutbestandteilen, pro zu untersuchende Probe bestimmt werden können. Des weiteren wird damit ein möglichst einfach herzustellendes und einfach, insbesondere mit wenigen Versuchsreihen und ohne Probenvorbereitung, zu handhabendes und kostengünstiges Testsystem bereitgestellt, mit dem gleichzeitig verschiedene zelluläre Parameter und/oder plasmatische Parameter einer Probe oder mehrerer zu untersuchender Proben, insbesondere Blutgruppenmerkmale, bestimmt werden können.

Die Membran der erfindungsgemäßen Vorrichtung ist eine poröse Membran. Bevorzugte Membran-Materialien sind beispielsweise Nitrozellulose (z. B. *UniSart* von Sartorius, *HiFlow* von Millipore, Whatman, *AE99* bzw. *FF85*/*100* von Schleicher & Schuell), Polyethylen (*Lateral Flo* von Porex Corporation) oder Nylon (*Novylon* von CUNO). Vorzugsweise weist die Membran eine möglichst große Porengröße auf, da eine hohe Porosität der Membran das Eindringen insbesondere von zellulären Komponenten der zu bestimmenden Probe, z. B. von Erythrozyten, in die poröse Struktur begünstigt. Von besonderem Vorteil ist der Einsatz aufnehmender Membranen. Die erfindungsgemäße Vorrichtung ist jedoch nicht auf diese Eigenschaften beschränkt. Bevorzugt werden alle Membranen mit einer hohen kapillaren Flussrate (Capillary Speed), wobei die kapillare Flussrate die Zeit ist, die eine Farblösung braucht, um 40 mm auf einer gegebenen Membran zurückzulegen. Besonders bevorzugt sind Membranen, deren kapillare Flussrate < 100 ist.

In einer bevorzugten Ausführungsform der Erfindung ist in Fließrichtung hinter der Aufgabezone und vor den Indikatorzonen der erfindungsgemäßen Vorrichtung auf der porösen Membran ein Dichtelement angeordnet. Zur Anwendung kommen zwei- oder dreidimensionale Dichtelemente, die auf der porösen Membran platziert werden und mit denen eine von der übrigen Fläche der porösen Membran separierte Probenauftragszone geschaffen wird. Das Dichtelement hat erfindungsgemäß primär die Wirkung einer Flüssigkeitsbarriere und erlaubt die gerichtete Verteilung von Probenflüssigkeit und Testreagenzien in die poröse Membran. Weiterhin dichtet das Dichtelement erfindungsgemäß die Probenauftragszone ab zur Verhinderung eines unerwünschten Flüssigkeitsübertritts in die anderen Bereiche der Lateral-Fluss-Vorrichtungsanordnung.

Bevorzugte Ausführungsformen des Dichtelementes sind die Steg- oder Trog- bzw. Trichter-Form. Die Ausformung des Dichtelementes erfolgt durch Schneideprozesse aus dem zur Herstellung des Dichtelementes verwendeten Material. Im Fall der Trichter- bzw. Trogform erhält das Dichtelement eine innere Öffnung, deren bevorzugte Ausführungsvarianten runde, quadratische oder rechteckige, im Fall der Trichterform sich zur Unterseite (Membrankontaktseite) des Dichtelementes verjüngende Formen sind.

Bevorzugte Materialien für das Dichtelement sind Materialien, die nicht wasseraufnehmend (hydrophob) sind. In einer besonderen Ausführungsform sind die Materialien einseitig mit einem Klebstofffilm, beispielsweise einem drucksensitiven bzw. selbsthaftenden Acrylatklebstoff, beschichtet. Somit kann das Dichtelement direkt auf die Oberfläche der porösen Membran geklebt werden. Alternativ kann das Dichtelement mit dem Lateral-Fluss-Gehäuse verbunden, beispielsweise verklebt sein, wobei in dieser Ausführungsform das Lateral-Fluss-Gehäuse das Dichtelement auf die Oberfläche der porösen Membran drückt und damit die Funktionen des Dichtelementes erzielt werden.

Bevorzugte Materialien für die Ausbildung von zweidimensionalen Dichtelementen sind jede Form von Klebebändern oder Klebefolien (z. B. Tesa 4124 von Beiersdorf AG, ARcare 7815 von Adhesives Research).

Bevorzugte Materialien für die Ausbildung von dreidimensionalen Dichtelementen sind flexible, geschlossenporige Elastomermaterialien oder flexible Silikonmaterialien mit unterschiedlichen Materialstärken, vorzugsweise 3-5 mm (z. B. Zellkautschuk EPDM140 von Pitzner, Silikonkautschuk oder Vollkautschuk, Härte 40° oder weniger, von Castan).

Durch diese erfindungsgemäße Ausgestaltung ist die erfindungsgemäße Vorrichtung in der Lage, flüssige Proben, die Zellen enthalten, wie beispielsweise Vollblut, aufzunehmen, ohne die Zellen dabei abzufiltern. Weiterhin erlaubt das Dichtelement das Auftragen großer Probenvolumina auf die poröse Membran (Aufgabezone), ohne dass diese überschwemmt wird. Somit unterstützt das Dichtelement die Nutzung der aufnehmenden Eigenschaften der porösen Membran. Weiter garantiert das Dichtelement einen gerichteten Probenfluss. Die erfindungsgemäße Vorrichtung kann jedoch mit oder ohne Dichtelement gut funktionieren.

Für den Absorptionsbereich (Absorptions-Pad) der erfindungsgemäßen Vorrichtung werden mechanisch stabile Materialien bevorzugt, vorzugsweise mit Wasserabsorptionskapazitäten von 20-30 g/100 cm² (z. B. Wicking Papier, Typ 300, Schleicher und Schüll). Der Kontakt zwischen dem Absorptions-Pad und der Lateral Fluss-Membran der erfindungsgemäßen Vorrichtung wird durch Andruck und Überlappung mit der porösen Membran hergestellt. Die genaue Positionierung des Absorptions-Pads auf der Membran wird durch Verkleben des Absorptions-Pads mit der, die Lateral Fluss-Membran tragenden Trägerschicht (backing sheet), erzielt.

In einer weiteren Ausführungsform sind die Komponenten der erfindungsgemäßen Vorrichtung zum Zwecke der mechanischen Verstärkung auf eine Unterlage bzw. Trägerschicht aufgebracht. Die erfindungsgemäße Vorrichtung kann jedoch mit oder ohne Trägerschicht funktionieren. Bevorzugt werden mechanisch stabile und nicht wasseraufnehmende Materialien, vorzugsweise mit Materialstärken von 100 µm oder mehr, die ein- oder zweiseitig mit einem Klebstofffilm, z. B. einem drucksensitiven bzw. selbsthaftenden Acrylatklebstoff, beschichtet sind (z.B. 0.005" Polyester W/ GL-187, G & L). Auf der Trägerschicht werden die poröse Membran und das Absorptions-Pad fixiert. Im Fall der doppelseitig klebenden Trägerschicht wird die klebende zweite Seite zur Fixierung des Stapels auf weiteren Flächen, z. B. innerhalb der Lateral-Fluss-Gehäuse, eingesetzt.

In einer weiteren Ausführungsform ist die erfindungsgemäße Vorrichtung, entweder mit oder ohne Trägerschicht, auf der die Komponenten der erfindungsgemäßen Vorrichtung aufgebracht sind, in einem Gehäuse integriert, wodurch die Membran-Komponenten aneinander gedrückt werden und das Gehäuse die Dichtelementfunktion unterstützt. Dabei kann die erfindungsgemäße Vorrichtung jedoch mit oder ohne Gehäuse genauso gut funktionieren.

Ein weiterer Gegenstand der Erfindung ist die Verwendung der erfindungsgemäßen Vorrichtung zur Analyse von Blut, insbesondere zur simultanen Bestimmung von Blutgruppenantigenen bzw. -antigen-Epitopen aller denkbaren Blutgruppensysteme, vorzugsweise jeglicher Analyten auf der Oberfläche der roten Blutkörperchen. Die zu testenden Antigene bzw. Antigen-Epitope sind beispielhaft solche des AB0-Blutgruppensystems des Rh-, Kell-, Lewis- Hh-, Duffy- Kidd, MNS-, Lutheran-, P-Systems, der Blutgruppensysteme Diego, Yt, Scianna, Dombrock, Colton, Chido/Rodgers, Gerbich, Cromer, Knops, Landsteiner-Wiener, Xg, Kx, Indian, Ok, Raph, John Milton Hagen, Langereis, und/oder Sid, insbesondere A1, A2, B, D, C, c, E, e, Cw, K, k, M, N, S, s, Jk(a), Jk(b), Fy(a), Fy(b), Kp(a), Kp(b), Js(a), Js(b), Le(a), Le(b), Lu(a), Lu(b), P1, I, H, Xg(a), U, Vw, Wr(a), Lan.

Eine bevorzugte Ausführungsform der erfindungsgemäßen Vorrichtung bestimmt gleichzeitig mehrere Blutgruppenmerkmale, beispielhaft A, B, AB, D, C, c, E, e, Cw und K. Die zu untersuchende Probe, beispielsweise natives oder antikoaguliertes Vollblut oder Erythrozytenkonzentrate oder verdünnte Erythrozyten-Suspensionen, wird auf die Aufgabezone der erfindungsgemäßen Vorrichtung aufgetragen. Die in der Probe enthaltenen Erythrozyten, die den/die Analyten tragen, dienen gleichzeitig als Indikatorpartikel.

Die Aufgabe wird erfindungsgemäß gelöst zum anderen durch ein Verfahren zur Bestimmung mehrerer Analyten oder deren Derivate in einer flüssigen Probe, welches das Auftragen der Probe auf die Aufgabezone einer Membran der erfindungsgemäßen Vorrichtung umfasst, wobei diese Probe in ausreichender Menge vorliegt, um die Probenflüssigkeit dazu zu veranlassen, in Richtung Absorptionsbereich durch die Indikatorzonen zu fließen und um die Analyten oder ihre Derivate in der Probenflüssigkeit dazu zu veranlassen, an die jeweiligen Indikatorzonen zu binden bzw. in den Indikatorzonen einen Komplex zu bilden.

Bei dem erfindungsgemäßen Verfahren handelt es sich bei den zu bestimmenden Analyten insbesondere um Blutgruppenantigene bzw. -antigen-Epitope sämtlicher Blutgruppensysteme, vorzugsweise solcher, die sich auf der Oberfläche der roten Blutkörperchen befinden. Die zu testenden Antigene bzw. Antigen-Epitope sind beispielhaft solche des AB0-Blutgruppensystems, des Rh-, Kell-, Lewis- Hh-, Duffy- Kidd, MNS-, Lutheran-, P-Systems, der Blutgruppensysteme Diego, Yt, Scianna, Dombrock, Colton, Chido/Rodgers, Gerbich, Cromer, Knops, Landsteiner-Wiener, Xg, Kx, Indian, Ok, Raph, John Milton Hagen, Langereis, und/oder Sid, insbesondere A1, A2, B, D, C, c, E, e, Cw, K, k, M, N, S, s, Jk(a), Jk(b), Fy(a), Fy(b), Kp(a), Kp(b), Js(a), Js(b), Le(a), Le(b), Lu(a), Lu(b), P1, I, H, Xg(a), U, Vw, Wr(a), Lan.

Eine bevorzugte Ausführungsform des erfindungsgemäßen Verfahrens bestimmt gleichzeitig mehrere Blutgruppenmerkmale, beispielhaft A, B, AB, D, C, c, E, e, Cw und K. Die zu untersuchende Probe, beispielsweise natives oder antikoaguliertes Vollblut oder Erythrozyten-Suspensionen mit oder ohne Testflüssigkeit, wie Kontrollblut, wird auf die Aufgabezone der erfindungsgemäßen Vorrichtung aufgetragen. Die in der Probe enthaltenen Erythrozyten, die den/die Analyten tragen, dienen gleichzeitig als Indikatorpartikel.

Im folgenden wird die Erfindung durch Figuren und Beispiele näher erläutert, ohne sie einzuschränken. Es zeigen:
- Fig. 1: eine perspektivische Darstellung einer erfindungsgemäßen Vorrichtung für Lateral-Fluss-Tests für die simultane Bestimmung der Blutgruppen merkmale A, B, AB, D und CDE;
- Fig. 2: eine Explosionsdarstellung der in Fig. 1 dargestellten erfindungsgemäßen Vorrichtung für Lateral-Fluss-Tests;
- Fig. 3: eine perspektivische Darstellung einer erfindungsgemäßen Vorrichtung für Lateral-Fluss-Tests für die simultane Bestimmung der Blutgruppen- merkmale A, B, AB, D und CDE, ausgeführt mit einem dreidimensiona- len Dichtelement in Stegform;
- Fig. 4: eine Explosionsdarstellung der in Fig. 3 dargestellten erfindungsgemäßen Vorrichtung für Lateral-Fluss-Tests;
- Fig. 5: eine perspektivische Darstellung einer erfindungsgemäßen Vorrichtung für Lateral-Fluss-Tests für die simultane Bestimmung der Blutgruppen- merkmale A, B, AB, D und CDE, ausgeführt mit einem dreidimensiona- len Dichtelement in Trogform;
- Fig. 6: eine Explosionsdarstellung der in Fig. 5 dargestellten erfindungsgemäßen Vorrichtung für Lateral-Fluss-Tests;
- Fig. 7: eine perspektivische Darstellung einer erfindungsgemäßen Vorrichtung für Lateral-Fluss-Tests für die simultane Bestimmung der Blutgruppen- merkmale A, B, AB, D, C, c, E, e, Cw und K;
- Fig. 8: eine perspektivische Darstellung einer erfindungsgemäßen Vorrichtung für Lateral-Fluss-Tests, ausgeführt als Bedside-Test zur Prüfung der AB0-Identität von Empfänger und Konserve;
- Fig. 9: eine Explosionsdarstellung der in Fig. 8 dargestellten erfindungsgemäßen Vorrichtung für Lateral-Fluss-Tests.
- Fig. 10: eine perspektivische Darstellung einer erfindungsgemäßen Vorrichtung für Lateral-Fluss-Tests für die simultane Bestimmung der Blutgruppen- merkmale A, B, AB, D und CDE.
- Fig. 11: eine perspektivische Darstellung einer erfindungsgemäßen Vorrichtung für Lateral-Fluss-Tests für die simultane Bestimmung der Blutgruppen- merkmale A, B, AB, D und CDE.
- Fig. 12: eine perspektivische Darstellung einer erfindungsgemäßen Vorrichtung für Lateral-Fluss-Tests für die simultane Bestimmung der Blutgruppen- merkmale A, B, AB, D und CDE.
- Fig. 13: eine perspektivische Darstellung einer erfindungsgemäßen Vorrichtung für Lateral-Fluss-Tests für die simultane Bestimmung der Blutgruppen- merkmale A, B, AB, D und CDE.
- Fig. 14: eine perspektivische Darstellung einer erfindungsgemäßen Vorrichtung für Lateral-Fluss-Tests mit bidirektionalem Fluss für die simultane Be- stimmung der Blutgruppenmerkmale A, B, AB, D und CDE;
- Fig. 15: eine Explosionsdarstellung der in Fig. 14 dargestellten erfindungsgemäßen Vorrichtung für Lateral-Fluss-Tests.

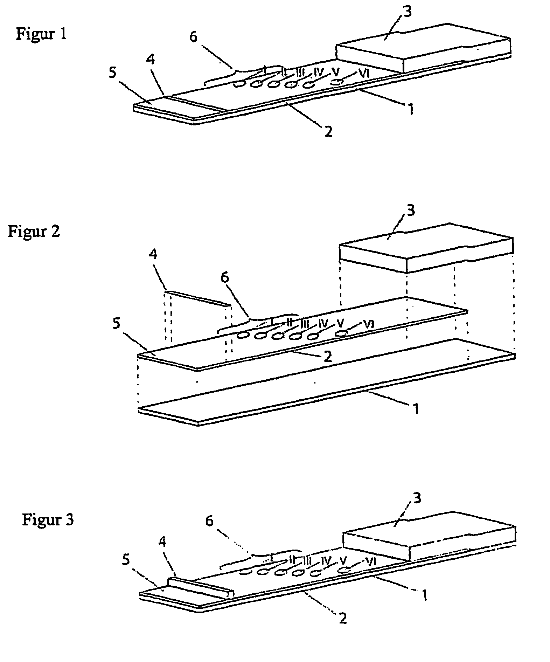

In Fig. 1 wird beispielhaft eine perspektivische Darstellung einer erfindungsgemäßen Vorrichtung für Lateral-Fluss-Tests für die simultane Bestimmung der Blutgruppenmerkmale A, B, AB, D und CDE gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I - VI gebildet, wobei die Indikatorzonen aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen.

In Fig. 2 wird eine Explosionsdarstellung der in Fig. 1 dargestellten erfindungsgemäßen Vorrichtung für Lateral-Fluss-Tests gezeigt, die aus den Komponenten Trägerschicht 1, poröse Membran 2, Absorptions-Pad 3 und Dichtelement 4 besteht, welches die Aufgabezone 5 von der übrigen Membran separiert, die wiederum den Indikatorzonenbereich 6 mit den von proximal nach distal diagonal versetzt angeordneten Indikatorzonen I-VI enthält.

In Fig. 3 wird beispielhaft eine perspektivische Darstellung einer erfindungsgemäßen Vorrichtung für Lateral-Fluss-Tests für die simultane Bestimmung der Blutgruppenmerkmale A, B, AB, D und CDE gezeigt. Im vorliegenden Beispiel entsprechen die Komponenten der Vorrichtung den Komponenten der in Fig. 1 dargestellten Vorrichtung mit Ausnahme des auf der Oberseite der porösen Membran 2 fixierten, in dreidimensionaler Stegform ausgeführten Dichtelements 4.

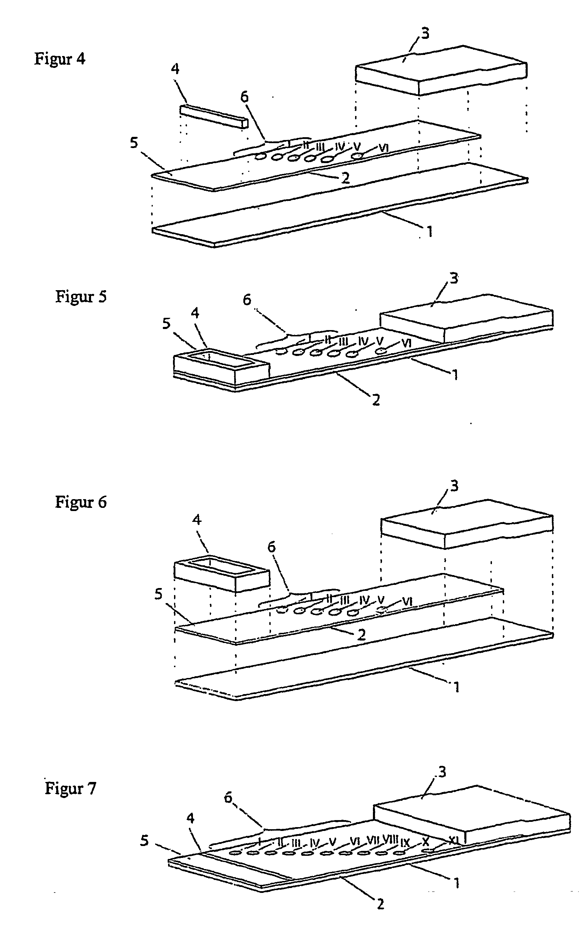

In Fig. 4 wird eine Explosionsdarstellung der in Fig. 3 dargestellten erfindungsgemäßen Vorrichtung für Lateral-Fluss-Tests mit den Komponenten Trägerschicht 1, poröse Membran 2, Absorptions-Pad 3 und in dreidimensionaler Stegform ausgeführtes Dichtelement 4 gezeigt, welches die Aufgabezone 5 von der übrigen Membran separiert, die wiederum den Indikatorzonenbereich 6 mit den von proximal nach distal diagonal versetzt angeordneten Indikatorzonen I-VI enthält.

In Fig. 5 wird beispielhaft eine perspektivische Darstellung einer erfindungsgemäßen Vorrichtung für Lateral-Fluss-Tests für die simultane Bestimmung der Blutgruppenmerkmale A, B, AB, D und CDE gezeigt. Im vorliegenden Beispiel entsprechen die Komponenten der Vorrichtung den Komponenten der in Fig. 1 dargestellten Vorrichtung mit Ausnahme des auf der Oberseite der porösen Membran 2 fixierten, in dreidimensionaler Trogform ausgeführten Dichtelements 4.

In Fig. 6 wird eine Explosionsdarstellung der in Fig. 5 dargestellten erfindungsgemäßen Vorrichtung für Lateral-Fluss-Tests mit den Komponenten Trägerschicht 1, poröse Membran 2, Absorptions-Pad 3 und in dreidimensionaler Trogform ausgeführtes Dichtelement 4 gezeigt, welches die Aufgabezone 5 von der übrigen Membran separiert, die wiederum den Indikatorzonenbereich 6 mit den von proximal nach distal diagonal versetzt angeordneten Indikatorzonen I-VI enthält.

In Fig. 7 wird beispielhaft eine perspektivische Darstellung einer erfindungsgemäßen Vorrichtung für Lateral-Fluss-Tests für die simultane Bestimmung der Blutgruppenmerkmale A, B, AB, D, C, c, E, e, Cw und K gezeigt. Im vorliegenden Beispiel besteht die Vorrichtung aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus diagonal versetzt, in definierten X- und Y-Positionen angeordneten, punktförmigen Indikatorzonen I - XI gebildet, wobei die Indikatorzonen aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-C (monoklonal) |
| VI | Antikörper | Anti-c (monoklonal) |
| VII | Antikörper | Anti-E (monoklonal) |
| VIII | Antikörper | Anti-e (monoklonal) |
| IX | Antikörper | Anti-Cw (monoklonal) |
| X | Antikörper | Anti-K (monoklonal) |
| XI | Antikörper | Anti-Erythrozyten (polyklonal) |

Indikatorzone XI ist die Kontrolle (ctl) und enthält polyklonale anti-Erythrozyten Antikörper. Sie befindet sich distal angeordnet zu allen übrigen Indikatorzonen.

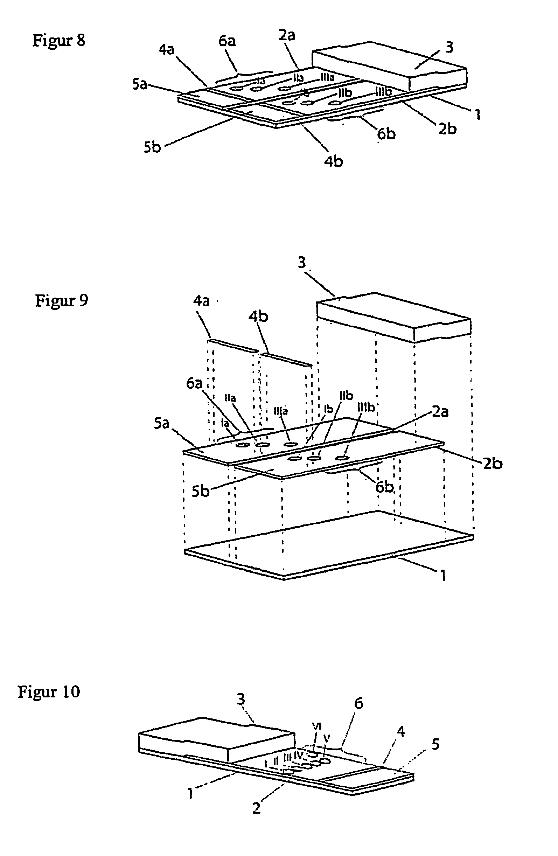

In Fig. 8 wird beispielhaft eine perspektivische Darstellung einer erfindungsgemäßen Vorrichtung für Lateral-Fluss-Tests, ausgeführt als Bedside-Test zur Prüfung der AB0-Identität von Empfänger und Konserve, gezeigt. Im vorliegenden Beispiel besteht die Vorrichtung aus einer Trägerschicht 1, der in doppelter Ausführung vorhandenen porösen Membran 2a und 2b, dem Absorptions-Pad 3 und den zweidimensionalen, in Stegform ausgeführten Dichtelementen 4a und 4b. Die beiden porösen Membranen 2a und 2b sind auf einer, mit einem drucksensitiven bzw. selbsthaftenden Acrylatklebstoff versehenen Trägerschicht 1 parallel und in gleicher Ausrichtung fixiert. Ebenso ist das Absorptions-Pad 3 auf der Trägerschicht 1 fixiert, wobei ein Teil des Absorptions-Pad 3 mit beiden porösen Membran 2a und 2b im gleichen Abstand überlappt Die auf der Oberseite der porösen Membranen 2a und 2b fixierten Dichtelemente 4a und 4b separieren die jeweiligen Aufgabezonen 5a und 5b von der übrigen Membranfläche und ermöglichen die gerichtete Verteilung von Probenflüssigkeit und Testreagenzien in die porösen Membranen 2a und 2b. Zwischen der Aufgabezone 5a bzw. 5b und den jeweiligen Bereichen der porösen Membranen 2a und 2b, die mit dem Absorptions-Pad 3 in Kontakt stehen, sind die Indikatorzonenbereiche 6a und 6b angeordnet. Diese werden aus diagonal versetzt, in definierten X- und Y-Positionen angeordneten, punktförmigen Indikatorzonen Ia - IIIa bzw. Ib - IIIb gebildet, wobei die Indikatorzonen aus den folgenden Bindungselementen bestehen:

| Indikatorzone | Bindungselement | Spezifikation |
|---|---|---|
| Ia, Ib | Antikörper | Anti-A (monoklonal) |
| IIa, IIb | Antikörper | Anti-B (monoklonal) |
| IIIa, IIIb | Antikörper | Anti-Erythrozyten (polyklonal) |

Indikatorzonen IIIa und IIIb sind die Kontrollen (ctl) und enthalten polyklonale anti-Erythrozyten Antikörper. Sie befinden sich distal angeordnet zu allen übrigen Indikatorzonen.

In Fig. 9 wird eine Explosionsdarstellung der in Fig. 8 dargestellten erfindungsgemäßen Vorrichtung für Lateral-Fluss-Tests mit den Komponenten Trägerschicht 1, poröse Membranen 2a und 2b, Absorptions-Pad 3 und den Dichtelementen 4a und 4b gezeigt, welche jeweils die Aufgabezonen 5a und 5b von der übrigen Membran separieren, die wiederum die Indikatorzonenbereiche 6a bzw. 6b mit den von proximal nach distal diagonal versetzt angeordneten Indikatorzonen Ia - IIIa und Ib - IIIb enthält.

In Fig. 10 wird beispielhaft eine perspektivische Darstellung einer erfindungsgemäßen Vorrichtung für Lateral-Fluss-Tests für die simultane Bestimmung der Blutgruppenmerkmale A, B, AB, D und CDE gezeigt. Das vorliegende Beispiel stellt eine Lateral-Fluss-Test Vorrichtung für Rechtshänder dar und besteht aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus parallel nebeneinander versetzt, in einer linearen Reihe angeordneten, in definierten X- und Y-Positionen angeordneten, punktförmigen Indikatorzonen I - VI gebildet, wobei die Indikatorzonen aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen.

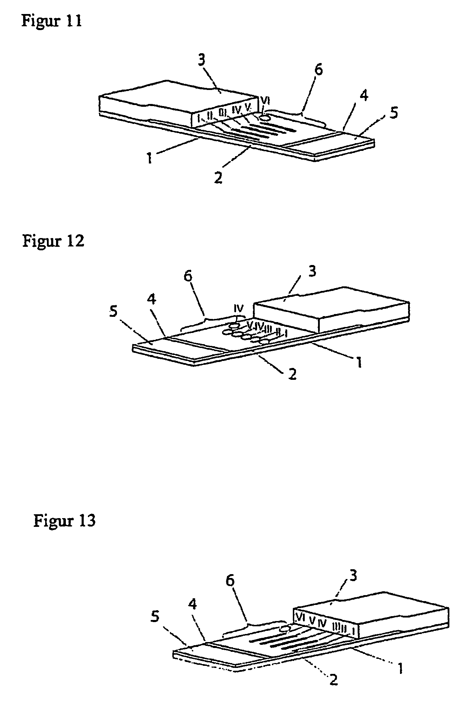

In Fig. 11 wird beispielhaft eine perspektivische Darstellung einer erfindungsgemäßen Vorrichtung für Lateral-Fluss-Tests für die simultane Bestimmung der Blutgruppenmerkmale A, B, AB, D und CDE gezeigt. Das vorliegende Beispiel stellt eine Lateral-Fluss-Test Vorrichtung für Linkshänder dar und besteht aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus parallel nebeneinander versetzt, in einer linearen Reihe angeordneten, in definierten X- und Y-Positionen angeordneten, punktförmigen Indikatorzonen I - VI gebildet, wobei die Indikatorzonen aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen.

In Fig. 12 wird beispielhaft eine perspektivische Darstellung einer erfindungsgemäßen Vorrichtung für Lateral-Fluss-Tests für die simultane Bestimmung der Blutgruppenmerkmale A, B, AB, D und CDE gezeigt. Das vorliegende Beispiel stellt eine Lateral-Fluss-Test Vorrichtung für Rechtshänder dar und besteht aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus parallel nebeneinander versetzt angeordneten, in definierten X- und Y-Positionen angeordneten, längsgezogenen bzw. bandförmigen Indikatorzonen I - VI gebildet, wobei die Indikatorzonen aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen.

In Fig. 13 wird beispielhaft eine perspektivische Darstellung einer erfindungsgemäßen Vorrichtung für Lateral-Fluss-Tests für die simultane Bestimmung der Blutgruppenmerkmale A, B, AB, D und CDE gezeigt. Das vorliegende Beispiel stellt eine Lateral-Fluss-Test Vorrichtung für Linkshänder dar und besteht aus einer Trägerschicht 1, der porösen Membran 2, dem Absorptions-Pad 3 und dem zweidimensionalen, in Stegform ausgeführten Dichtelement 4. Dabei ist die poröse Membran 2 auf der mit einem drucksensitiven bzw. selbsthaftenden Acrylatklebstoff versehenen Trägerschicht 1 fixiert. Ebenso ist das Absorptions-Pad 3 auf der Trägerschicht 1 fixiert, wobei ein Teil des Absorptions-Pad 3 mit der porösen Membran 2 überlappt. Das auf der Oberseite der porösen Membran 2 fixierte Dichtelement 4 separiert die Aufgabezone 5 von der übrigen Membranfläche und ermöglicht die gerichtete Verteilung von Probenflüssigkeit und Testreagenzien in die poröse Membran 2. Zwischen der Aufgabezone 5 und dem Bereich der porösen Membran 2, der mit dem Absorptions-Pad 3 in Kontakt steht, ist der Indikatorzonenbereich 6 angeordnet. Dieser wird aus parallel nebeneinander versetzt angeordneten, in definierten X- und Y-Positionen angeordneten, längsgezogenen bzw. bandförmigen Indikatorzonen I - VI gebildet, wobei die Indikatorzonen aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VI | Antikörper | Anti-Erythrozyten (polyklonal) |

Indikatorzone VI ist die Kontrolle (ctl) und enthält polyklonale anti-Erythrozyten Antikörper. Sie befindet sich distal angeordnet zu allen übrigen Indikatorzonen.

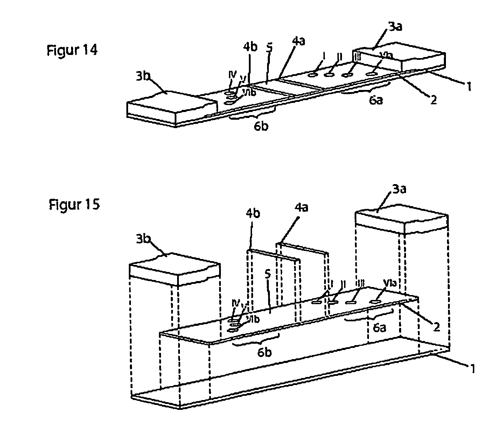

In Fig. 14 wird beispielhaft eine perspektivische Darstellung einer erfindungsgemäßen Vorrichtung für Lateral-Fluss-Tests mit bidirektionalem Fluss für die simultane Bestimmung der Blutgruppenmerkmale A, B, AB, D und CDE gezeigt. Im vorliegenden Beispiel besteht die Vorrichtung aus einer Trägerschicht 1, der porösen Membran 2, den Absorptions-Pads 3a und 3b und den zweidimensionalen, in Stegform ausgeführten Dichtelementen 4a und 4b. Dabei ist die poröse Membran 2 auf der mit einem drucksensitiven Acrylatklebstoff versehenen Trägerschicht 1 fixiert. Ebenso sind die Absorptions-Pads 3a und 3b auf der Trägerschicht 1 fixiert, wobei ein Teil der Absorptions-Pads 3a und 3b mit der porösen Membran 2 überlappt. Die auf der Oberseite der porösen Membran 2 fixierten Dichtelemente 4a und 4b separieren die in der Mitte der Membran liegende Aufgabezone 5 von der übrigen Membranfläche und ermöglichen die gerichtete bidirektionale Verteilung von Probenflüssigkeit und Testreagenzien in die poröse Membran 2. Zwischen der Aufgabezone 5 und dem Bereich der porösen Membran 2, der mit den Absorptions-Pads 3a und 3b in Kontakt steht, sind die Indikatorzonenbereiche 6a und 6b angeordnet. Diese werden aus diagonal versetzten, in definierten X- und Y-Positionen angeordneten, punktförmigen Indikatorzonen I - VI gebildet, wobei die Indikatorzonen aus den folgenden Bindungselementen bestehen:

| Indikatorzone | Bindungselement | Spezifikation |
|---|---|---|
| I | Antikörper | Anti-A (monoklonal) |
| II | Antikörper | Anti-B (monoklonal) |
| III | Antikörper | Anti-AB (monoklonal) |
| IV | Antikörper | Anti-D (monoklonal) |
| V | Antikörper | Anti-CDE (monoklonal) |
| VIa, VIb | Antikörper | Anti-Erythrozyten (polyklonal) |

Die Indikatorzonen VIa und VIb sind die Kontrollen (ctl) und enthalten polyklonale anti-Erythrozyten Antikörper. Sie befinden sich distal angeordnet zu den Indikatorzonen I-III bzw. IV-V.

In Fig. 15 wird eine Explosionsdarstellung der in Fig. 14 dargestellten erfindungsgemäßen Vorrichtung für Lateral-Fluss-Tests mit bidirektionalem Fluss gezeigt, die aus den Komponenten Trägerschicht 1, poröse Membran 2, Absorptions-Pads 3a und 3b und den Dichtelementen 4a und 4b bestehen, welche die mittig liegende Aufgabezone 5 von der übrigen Membranfläche separiert, die wiederum zwei Indikatorzonenbereiche 6a und 6b mit den von proximal nach distal diagonal versetzt angeordneten Indikatorzonen I, II, III, VIa bzw. IV, V, VIb enthalten.

### Beispiele

### Beispiel 1: Blutgruppenbestimmung

### Herstellung der Teststreifen:

Die Teststreifen bestehen aus einer Aufgabezone, einem Indikatorzonenbereich und einem Absorptionsbereich. Membranen der Sorte Millipore HiFlow Plus 065 werden in Streifen auf eine Größe von 15 x 35 mm (Breite/Länge; x/y) für eine 6-Spot- Ausführung, bzw. auf eine Größe von 26 x 40 mm für eine 11-Spot-Ausführung zurechtgeschnitten und auf eine Trägerschicht (Backing Sheet z. B. von G&L) aufgeklebt. Diagonal versetzt oder alternativ in einer linearen Reihe versetzt werden im Indikatorzonenbereich 0,2 µl Punkte von Lösungen verschiedener blutgruppenspezifischer monoklonaler Antikörper unter Verwendung eines Dispersens, z. B. AD3200 (Biodot), aufgetragen:

Anti-A-Klon Birma-1 (Serologicals, TLJ0105); Anti-B-Klon ES-4 (Serologicals, NCA0201); Anti-AB-Klone AB6, AB26, AB92 (Medion Diagnostics, 010062); Anti-D-Klon LDM3 (SNBTS, Z7180100); Anti-C-Klon MS-24 (Serologicals, un-formulated, KGK0212); Anti-c-Klon MS-33 (Serologicals, KNI0207); Anti-E-Klons MS-80 + MS-258 (Serologicals, KXE0201); Klons Anti-e MS-21+MS-63 (Serologicals, KLL0205+KQK0205); Anti-Cw-Klon MS-110 (Serologicals, JPK0201); Anti-K-Klon MS-56, (Serologicals, KOA0201).

Die Positionierung des anti-A Antikörpers erfolgt in Position x=3/y=10 mm. Alle anderen Antikörper werden iterierend in Abständen von x=1,5/y=2,2 mm zur Position des anti-A Antikörpers dispensiert. Der anti-Erythrozyten-spezifische Kontrollantikörper (Rabbit IgG Fraction of anti Human RBC, Rockland, 209-4139) wird in x=2/y=3,5 mm Versetzung zum letzten Spot der Serie der blutgruppenspezifischen Antikörper aufgetragen. Die Verdünnungen der Antikörper erfolgen in 15 mM Kaliumphosphatpuffer pH 7,5, 10% (v/v) Methanol) wie folgt: Anti-A Antikörper 1:3, anti-B Antikörper 1:2, anti-AB Antikörper 1:4, anti-D Antikörper 1:4, anti-RBC Antikörper 1:3. Alle anderen Antikörperlösungen werden nicht vorverdünnt, jedoch mit Methanol auf 10% (v/v) versetzt.

Die Membranen werden nach dem Dispensieren der Antikörper für 20 min bei 40°C getrocknet und anschließend bei konstanter Luftfeuchte bis zur Testdurchführung aufbewahrt. Am zur Aufgabezone distalen Ende wird ein mit der Membran um 3 mm überlappendes 15x10 mm bzw. 26x10 mm großes Absorptions-Pad (Schleicher & Schüll, 300) aufgeklebt. Die Aufgabezone wird durch Aufkleben eines 1-2 mm breiten Klebestreifens (Tesa 4124) in Position y=5 mm über die gesamte Membranbreite von der übrigen Membran separiert.

### Testansatz:

Als Blutproben werden antikoagulierte Vollblute verwendet. Für den eigentlichen Test werden 100 µl 1:6 in Verdünnungspuffer (EnlisstII, Medion Diagnostics oder Diluent 1, DiaMed) verdünntes Blut (6-Spot-Ausführung) bzw. 150 µl (11-Spot-Ausführung) in die Aufgabezone aufgetragen. Wenn das Blut die Aufgabezone verlassen hat, werden einmalig 100 µl bzw. 150 µl Verdünnungspuffer oder vorzugsweise 100 µl hypoosmotischer Waschpuffer (15 mM Kaliumphosphatpuffer pH 7,4, 0,3-0,45 % (w/v) NaCl) auf die Aufgabenzone pipettiert, um ungebundene Erythrozyten aus der Membran zu waschen. Alternativ kann der Probenauftrag aber auch mit 50 µl 1:3 verdünntem oder unverdünntem Blut erfolgen. Bei diesen Proben wird die Membran zweimalig mit Verdünnungspuffer bzw. einmalig mit Verdürmungspuffer und nachfolgend mit hypoosmotischem Waschpuffer gewaschen.

Bei der gewählten 1:6 Verdünnung ist die anti-RBC Kontrolle als Indikator eines erfolgreich durchgeführten Tests nach 2 Minuten sichtbar. Mit unverdünntem Blut dauert der Test länger.

### Ergebnis:

Der Test ist valid, wenn die anti-RBC Kontrolle ein deutlich positives Signal (Roter Punkt) zeigt. Je nach Anwesenheit oder Abwesenheit der jeweiligen Blutgruppenantigene erscheinen an den entsprechenden Positionen rote Punkte (positiv) oder die fast weisse Hintergrundfärbung der Membran (negativ).

### Beispiel 2: Bedside Test

### Herstellung der Teststreifen:

Der Bedside-Test besteht aus je zwei, auf einer Trägerschicht (Backing Sheet) fixierten Membranen ("Konserve", "Empfänger"), die jeweils aus einer Aufgabezone, einem Indikatorzonenbereich und einem Absorptionsbereich bestehen.

Membranen der Sorte Millipore HiFlow Plus 065 werden in Streifen auf eine Größe von 12,5 x 30 mm (Breite/Länge; x/y) zurechtgeschnitten. Jeweils zwei davon werden auf eine Trägerschicht (Backing Sheet z. B. von G&L) im Abstand von 5 mm aufgeklebt, so dass das Gesamt-Assembly eine Größe von 30 x 30 mm hat.

Diagonal versetzt werden auf beide Membranen unter Verwendung eines Dispensers, Z. B. AD3200 (Biodot) folgende, jeweils dieselben Auftragungen vorgenommen: 0,2 µl Punkte von Lösungen der monoklonalen Antikörper Anti-A-Klon Birma-1 (Serologicals, TLJ0105) in Position x=4/y=12 mm; Anti-B-Klon ES-4 (Serologicals, NCA0201) in Position x=7/y=14 mm. Der anti-Erythrozyten-spezifische Kontrollantikörper (Rabbit IgG Fraction of anti Human RBC, Rockland, 209-4139) wird in x=3/y=4 mm Versetzung zum anti-B Spot aufgetragen. Die Verdünnungen der Antikörper erfolgen in 15 mM Kaliumphosphatpuffer pH 7,5, 10% (v/v) Methanol) wie folgt: Anti-A Antikörper 1:3, anti-B Antikörper 1:2, anti-RBC Antikörper 1:3.

Die Membranen werden nach dem Dispensieren der Antikörper für 20 min bei 40°C getrocknet und anschließend bei konstanter Luftfeuchte bis zur Testdurchführung aufbewahrt. Am zur Aufgabenzone distalen Ende wird ein mit beiden Membranen um 3 mm überlappendes 30x10 mm großes Absorptions-Pad (Schleicher & Schüll, 300) aufgeklebt. Die Aufgabezone wird durch Aufkleben eines 1-2 mm breiten Klebestreifens je Teststreifen (Tesa 4124) in Position y=5 mm über die gesamte Membranbreite von der übrigen Membran separiert.

### Testansatz:

Als Proben werden verwendet: Für die Membran "Konserve": Erythrozytenkonzentrat; für die Membran "Empfanger": Vollblut.

Für den eigentlichen Test werden 50 µl Vollblut auf der Seite "Empfänger" und 50 µl Erythrozytenkonzentrat auf der Seite "Konserve" in die jeweilige Aufgabezone aufgetragen. Nachdem das Blut von der Membran ganz aufgesogen worden ist, wird mit jeweils 2 x 100 µl Verdünnungspuffer bzw. einmalig mit Verdünnungspuffer und nachfolgend mit hypoosmotischem Waschpuffer gewaschen.

### Ergebnis:

Die anti-RBC Kontrolle als Indikator eines erfolgreich durchgeführten Tests wird bei beiden Membranen nach etwa 2 Minuten sichtbar.

Der Test ist valid, wenn die anti-RBC Kontrolle ein deutlich positives Signal (roter Punkt) zeigt. Je nach Anwesenheit oder Abwesenheit der jeweiligen Blutgruppenantigene erscheinen an den entsprechenden Positionen rote Punkte (positiv) oder die fast weiße Hintergrundfärbung der Membran (negativ). Ein identisches Bild für "Empfänger" und "Konserve" bedeutet ABO-Identität zwischen Empfänger und Konserve.

### Beispiel 3: Blutgruppenbestimmung mit bidirektionalem Lateral-Fluss-Test

### Herstellung der Teststreifen:

Die Teststreifen bestehen aus einer in der Mitte der Membran liegenden Aufgabezone, zwei Indikatorzonenbereichen und zwei Absorptionsbereichen. Membranen der Sorte Millipore HiFlow Plus 065 werden in Streifen auf eine Größe von 15 x 50 mm (Breite/Länge, x/y) zurechtgeschnitten und auf eine Trägerschicht (Backing Sheet z. B. von G&L) aufgeklebt. Diagonal versetzt oder alternativ in einer linearen Reihe versetzt werden im Indikatorzonenbereich 0,2 µl Punkte von Lösungen verschiedener blutgruppenspezifischer monoklonaler Antikörper aufgetragen. Dabei ist die Mitte der Streifenlänge (y=0 mm) die Bezugsgröße für die Positionierung der Indikatorzonen in y-Richtung. Folgende Antikörper werden unter Verwendung eines Dispensers, z. B. AD3200 (Biodot), dispensiert:
   Anti-A-Klon Birma-1 (Serologicals, TLJ0105); Anti-B-Klon ES-4 (Serologicals, NCA0201); Anti-AB-Klone AB6, AB26, AB92 (Medion Diagnostics, 010062); Anti-D-Klon LDM3 (SNBTS, Z7180100); Anti-C-Klon MS-24 (Serologicals, un-formulated, KGK0212); Anti-E-Klone MS-80 + MS-258 (Serologicals, KXE0201). Für die anti-CDE Indikatorzone werden die anti-D und anti-C Antikörper zweifach, die anti-E Antikörper dreifach aufkonzentriert und in gleichen Volumenteilen gemischt.

In der Ausführungsform der diagonalen Versetzung der Indikatorzonen erfolgt die Dispensierung des anti-A Antikörpers in Position x=4/y=10 mm. Die Positionen der anti-B und anti-AB Antikörper erfolgt iterierend in Abständen von x=3,5/y=2 mm zur Position des anti-A Antikörpers. Der anti-Erythrozyten-spezifische Kontrollantikörper (Rabbit IgG Fraction of anti Human RBC, Rockland, 209-4139) wird in x=3,5/y=3,5 mm Versetzung zum letzten Spot der Serie der anti-A, anti-B und anti-AB Antikörper aufgetragen. Die Dispensierung des anti D-Antikörpers erfolgt in Position x=4/y=-10 mm, die Dispensierung der anti-CDE Antikörper im Abstand von x=3,5/y=-2 mm. Der anti-Erythrozyten-spezifische Kontrollantikörper wird in x=3,5/y=-3,5 mm Versetzung zum Spot der anti-CDE Antikörper aufgetragen. Die Verdünnungen der Antikörper erfolgen in 15 mM Kaliumphosphatpuffer pH 7,5, 10% (v/v) Methanol) wie folgt: Anti-A Antikörper 1:3, anti-B Antikörper 1:2, anti-AB Antikörper 1:4, anti-D Antikörper 1:3, anti-RBC Antikörper 1:3. Die Antikörpermischung anti-CDE wird nicht vorverdünnt, jedoch mit Methanol auf 10% (v/v) versetzt.

Die Membranen werden nach dem Dispensieren der Antikörper für 20 min bei 40°C getrocknet und anschließend bei konstanter Luftfeuchte bis zur Testdurchführung aufbewahrt. An den zur Aufgabenzone distalen Enden der Membran werden zwei mit der Membran um 3 mm überlappende 15x10 mm große Absorptions-Pads (Schleicher Schüll, 300) aufgeklebt. Die Aufgabezone wird durch Aufkleben von zwei 1-2 mm breiten Klebestreifen (Tesa 4124) in Position y=3 mm bzw. y=-3 mm über die gesamte Membranbreite von der übrigen Membran separiert.

### Testansatz:

Als Blutproben werden antikoagulierte Vollblute verwendet. Für den eigentlichen Test werden 100 µl 1:6 in Verdünnungspuffer (EnlisstII, Medion Diagnostics) verdünntes Blut in die Aufgabezone aufgetragen. Wenn das Blut die Aufgabezone verlassen hat, werden einmalig 100 µl Verdünnungspuffer oder 100 µl hypoosmotischer Waschpuffer (15 mM Kaliumphosphatpuffer pH 7,4, 0,3-0,45 % (w/v) NaCl) auf die Aufgabenzone pipettiert, um ungebundene Erythrozyten aus der Membran zu waschen. Alternativ kann der Probenauftrag aber auch mit 50 µl 1:3 verdünntem oder unverdünntem Blut erfolgen. Bei diesen Proben wird die Membran zweimalig mit Verdünnungspuffer bzw. einmalig mit Verdünnungspuffer und nachfolgend mit hypoosmotischen Waschpuffer gewaschen.

Bei der gewählten 1:6 Verdünnung ist die anti-RBC Kontrolle als Indikator eines erfolgreich durchgeführten Tests nach 2 Minuten sichtbar. Mit unverdünntem Blut dauert der Test länger.

### Ergebnis:

Der Test ist valid, wenn die anti-RBC Kontrolle ein deutlich positives Signal (Roter Punkt) zeigt. Je nach Anwesenheit oder Abwesenheit der jeweiligen Blutgruppenantigene erscheinen an den entsprechenden Positionen rote Punkte (positiv) oder die fast weisse Hintergrundfärbung der Membran (negativ).

## Patentansprüche

1. Vorrichtung zum gleichzeitigen, qualitativen oder quantitativen Bestimmen mehrerer Analyten in einer flüssigen Probe, wobei die Analyten Blutgruppenantigene bzw. -antigen-Epitope sind, umfassend eine Membran (2) mit
- einer Aufgabezone (5) zum Auftragen der flüssigen Probe,
- mindestens zwei Indikatorzonen, die mit dem/den Analyten in Wechselwirkung treten können und
- mindestens einem Absorptionsbereich (3), welcher die Flüssigkeit nach Passieren der Indikatorzonen aufnimmt,
wobei die Indikatorzonen zwischen der Aufgabezone (5) und einem Absorptionsbereich (3) liegen, **dadurch gekennzeichnet, dass**
die Fließrichtungen von der Aufgabezone (5) durch die jeweiligen Indikatorzonen zu einem Absorptionsbereich (3) (Fließspuren) im Wesentlichen parallel sind und mindestens zwei unterschiedliche Fließspuren vorliegen, wobei die Indikatorzonen so angeordnet sind, dass die Probenflüssigkeit pro Fließspur nicht mehr als eine Indikatorzone durchströmt, und die Indikatorzonen Antikörper bzw. Antikörperfragmente gegen Blutgruppenantigene und/oder Lektine bzw. Fragmente davon gegen Blutgruppenantigene umfassen.

2. Vorrichtung nach Anspruch 1, wobei die Indikatorzonen in einer diagonalen, V-, W-, M-, N-förmigen oder linearen Reihe angeordnet sind.

3. Vorrichtung nach einem der Ansprüche 1 bis 2, wobei die Indikatorzonen insbesondere anti-A, -B, -AB, -D, -C, -c, -E, -e, -Cw, und/oder -K-Antikörper bzw. Antikörperfragmente umfassen.

4. Vorrichtung nach einem der Ansprüche 1 bis 3, wobei die Membran (2) vorzugsweise aus Polyethylen, Nitrozellulose oder Nylon, besteht.

5. Vorrichtung nach einem der Ansprüche 1 bis 4, wobei hinter der Aufgabezone (5) und vor den Indikatorzonen auf der Membran (2) mindestens ein Dichtelement (4) angeordnet ist.

6. Vorrichtung nach einem der Ansprüche 1 bis 5, wobei die Komponenten der Vorrichtung zur mechanischen Verstärkung auf eine Trägerschicht (1) aufgebracht sind.

7. Vorrichtung nach einem der Ansprüche 1 bis 6, wobei die Komponenten der Vorrichtung in einem Gehäuse integriert sind.

8. Verwendung der Vorrichtung nach einem der Ansprüche 1 bis 7 zur Analyse von Blut, insbesondere zur simultanen Bestimmung der A-, B-, AB-, D-, C-, c-, E-, e-, Cw- und/oder K-Blutgruppenantigene bzw. -antigen-Epitope.

9. Verfahren zur Bestimmung mehrerer Analyten oder deren Derivate in einer flüssigen Probe, wobei die Analyten Blutgruppenantigene bzw. -antigen-Epitope sind, umfassend:
das Auftragen der Probe auf die Aufgabezone (5) einer Membran (2) der Vorrichtung nach einem der vorangegangenen Ansprüche 1 bis 8, wobei diese Probe in ausreichender Menge vorliegt, um die Probenflüssigkeit dazu zu veranlassen, in Richtung Absorptionsbereich (3) durch die Indikatorzonen zu fließen und um die Analyten oder ihre Derivate in der Probenflüssigkeit dazu zu veranlassen, in den Indikatorzonen einen Komplex zu bilden.

10. Verfahren nach Anspruch 9, wobei die Analyte insbesondere A-, B-, AB-, D-, C-, c-, E-, e-, Cw- und/oder K-Blutgruppenantigene bzw. -antigen-Epitope umfassen.

11. Verfahren nach einem der Ansprüche 9 bis 10, wobei die Analyte A-, B-, AB-, D-, C-, c-, E-, e-, Cw- und K-Blutgruppenantigene bzw. -antigen-Epitope gleichzeitig bestimmt werden.

12. Verfahren nach einem der Ansprüche 9 bis 11, wobei die Indikatorpartikel Erythrozyten sind.

13. Verfahren nach einem der Ansprüche 9 bis 12, wobei die Membran (2) nach Auftragung der Indikatorpartikel gewaschen wird.

14. Verfahren nach Anspruch 13, wobei der Waschpuffer vorzugsweise hypoosmotisch ist.

15. Verfahren nach einem der Ansprüche 9 bis 14, wobei die flüssige Probe aus Blut oder Blutbestandteilen, vorzugsweise aus Vollblut, ErythrozytenKonzentrat, oder Testflüssigkeit, wie Kontrollblut, besteht.

## Claims

1. Device for the simultaneous and qualitative or quantitative determination of a plurality of analytes in a liquid sample, wherein the analytes are blood group antigens or antigen epitopes, comprising a membrane (2) with
- an application zone (5) for the application of the liquid sample,
- at least two indicator zones, which are able to interact with the analyte(s) and
- at least one absorption region (3), which takes up the liquid after passage of the indicator zones,
wherein the indicator zones are located between the application zone (5) and the absorption region (3), **characterized in that** the flow directions from the application zone (5) through the respective indicator zones towards an absorption region (flow tracks) are substantially parallel and that at least two different flow tracks are present,
wherein the indicator zones are arranged such that the sample liquid per flow track does not flow through more than one indicator zone, and the indicator zones comprise antibodies or antibody fragments against blood group antigens and/or lectines, or fragments thereof against blood group antigens.

2. Device according to claim 1, wherein the indicator zones are arranged in a diagonal V-, W-, M-, N-shaped or linear row.

3. Device according to any one of claims 1 to 2, wherein the indicator zones comprise anti-A, -B, -AB, -D, -C, -c, E, -e, -Cw and/or K-antibodies or antibody fragments.

4. Device according to any one of claims 1 to 3, wherein the membrane (2) consists of polyethylene, nitrocellulose or nylon.

5. Device according to any one of claims 1 to 4, wherein downstream of the application zone (5) and upstream of the indicator zones at least one sealing element (4) is arranged on the membrane (2).

6. Device according to any one of claims 1 to 5, wherein the components of the device are applied onto a support layer (1) for mechanical reinforcement.

7. Device according to any one of claims 1 to 6, wherein the components of the device are integrated in a casing.

8. Use of the device according to any one of claims 1 to 7 for the analysis of blood, in particular for the simultaneous determination of A-, B-, AB-, D-, C-, c-, E-, e-, Cw- and/or K-blood group antigens or antigen epitopes.

9. Method for the determination of a plurality of analytes or their derivatives in a liquid sample, wherein the analytes are blood group antigens or antigen epitopes, comprising:
the application of the sample onto the application zone (5) of a membrane (2) of the device according to any one of the preceding claims 1 to 8, wherein this sample is present in adequate amounts in order to induce the test liquid to flow in the direction of the absorption region (3) through the indicator zones and to induce the analytes or their derivatives in the test liquid to form a complex in the indicator zones.

10. Method according to claim 9 , wherein the analytes include A-, B-, AB-, D-, C-, c-, E-, e-, Cw- and/or K-blood group antigens or antigen epitopes.

11. Method according to any one of claims 9 to 10, wherein the analytes A-, B-, AB-, D-, C-, c-, E-, e-, Cw- and/or K-blood group antigens or antigen epitopes are detected simultaneously.

12. Method according to any one of claims 9 to 11, wherein the indicator particles are erythrocytes.

13. Method according to any one of claims 9 to 12, wherein the membrane is rinsed after the application of indicator particles.

14. Method according to claim 13 wherein the washing buffer is hypo-osmotic.

15. Method according to any one of claims 9 to 14, wherein the liquid sample is composed of blood or blood components, preferably of whole blood, erythrocyte concentrate or test liquid such as control blood.

## Revendications

1. Dispositif pour la détermination qualitative ou quantitative simultanée de plusieurs analytes dans un échantillon liquide, les analytes étant des antigènes de groupes sanguins ou des épitopes d'antigènes de groupes sanguins, comprenant une membrane (2), qui comprend
- une zone de dépôt (5) pour application de l'échantillon liquide,
- au moins deux zones indicatrices, qui peuvent entrer en interaction avec le/ou les analyte(s), et
- au moins un domaine d'absorption (3), qui reçoit le liquide après passage par les zones indicatrices,
les zones indicatrices étant situées entre la zone de dépôt (5) et un domaine d'absorption (3), **caractérisé en ce que** les sens d'écoulement, à partir de la zone de dépôt (5), passant par les différentes zones indicatrices, jusqu'à un domaine d'absorption (3) (traces d'écoulement) sont pour l'essentiel parallèles, et que sont présentes au moins deux traces d'écoulement différentes, les zones indicatrices étant disposées de telle sorte que le liquide échantillon, pour chaque trace d'écoulement, ne passe pas par plus d'une zone indicatrice, et les zones indicatrices comprennent des anticorps ou des fragments d'anticorps contre des antigènes des groupes sanguin et/ou des lectines ou des fragments de ces derniers, contre des antigènes des groupes sanguins.

2. Dispositif selon la revendication 1, dans lequel les zones indicatrices sont disposées selon une rangée diagonale, en V, en W, en M, en N, ou linéaire.

3. Dispositif selon l'une des revendications 1 à 2, dans lequel les zones indicatrices comprennent en particulier des anticorps ou des fragments d'anticorps anti-A, -B, -AB, -D, -C, -c, -E, -e, -Cw et/ou -K.

4. Dispositif selon l'une des revendications 1 à 3, dans lequel la membrane (2) est de préférence constituée de polyéthylène, de nitrocellulose ou de nylon.

5. Dispositif selon l'une des revendications 1 à 4, dans lequel au moins un élément d'étanchéité (4) est disposé en arrière de la zone de dépôt (5) et en avant des zones indicatrices sur la membrane (2).

6. Dispositif selon l'une des revendications 1 à 5, dans lequel les composants du dispositif sont, pour un renforcement mécanique, appliqués sur une couche formant support (1).

7. Dispositif selon l'une des revendications 1 à 6, dans lequel les composants du dispositif sont intégrés dans un boîtier.

8. Utilisation du dispositif selon l'une des revendications 1 à 7 pour l'analyse du sang, en particulier pour la détermination simultanée des antigènes ou des épitopes des antigènes des groupes sanguins A, B, AB, D, C, c, E, e, Cw et/ou K.

9. Procédé pour déterminer plusieurs analytes ou leurs dérivés dans un échantillon liquide, les analytes étant des antigènes ou des épitopes d'antigènes des groupes sanguins, comprenant :
l'application de l'échantillon sur la zone de dépôt (5) d'une membrane (2) du dispositif selon l'une des revendications précédentes 1 à 8, cet échantillon étant présent en une quantité suffisante pour amener le liquide échantillon à s'écouler dans la direction du domaine d'absorption (3) en traversant les zones indicatrices, et pour amener les analytes ou leurs dérivés se trouvant dans le liquide échantillon à former un complexe dans les zones indicatrices.

10. Procédé selon la revendication 9, dans lequel les analytes comprennent en particulier des antigènes ou des épitopes d'antigènes des groupes sanguins A, B, AB, D, C, c, E, e, Cw et/ou K.

11. Procédé selon l'une des revendications 9 à 10 dans lequel on détermine simultanément les analytes antigènes ou épitopes d'antigènes des groupes sanguins A, B, AB, D, C, c, E, e, Cw et K.

12. Procédé selon l'une des revendications 9 à 11, dans lequel les particules indicatrices sont des érythrocytes.

13. Procédé selon l'une des revendications 9 à 12, dans lequel la membrane (2) est lavée après application des particules indicatrices.

14. Procédé selon la revendication 13, dans lequel le tampon de lavage est de préférence hypo-osmotique.

15. Procédé selon l'une des revendications 9 à 14, dans lequel l'échantillon sanguin est constitué de sang ou de constituants du sang, de préférence de sang total, d'un concentré érythrocytaire ou d'un liquide d'essai, tel qu'un sang témoin.
